# EUROPEAN PATENT APPLICATION

(11) **EP 4 191 324 A1**
(43) Date of publication of application: **07.06.2023**
(21) Application number: 21854216.5
(22) Date of filing: 02.08.2021
(51) Int. Cl.: G02C 7/04, G02C 13/00

(54) **CONTACT LENS SOLUTION AND UTILIZATION THEREOF**

(30) Priority: 03.08.2020 JP 2020131547
(71) Applicant: Seed Co., Ltd., Tokyo 113-8402 (JP)
(72) Inventor: OTAKE, Hideyuki, Tokyo 113-8402 (JP); JONIN, Kunio, Tokyo 113-8402 (JP)
(74) Representative: Schulz Junghans Patentanwälte PartGmbB
(86) International application number: PCT/JP2021/028534
(87) International publication number: WO 2022/030421

(57) **Abstract**

The objective of the present invention is to provide a contact lens solution that can durably reduce friction on the surface of a contact lens, such as hydrogel contact lens and that can be used to obtain a contact lens having surface smoothness. The objective is achieved by a contact lens solution containing a nonionic surfactant and a polysaccharide; a method for smoothing the surface of a contact lens, including bringing the contact lens solution into contact with the contact lens; and the like.

## Description

### Technical Field

The present invention relates to a contact lens solution. In more detailed, the present invention relates to a contact lens solution for reducing friction on the surface of the contact lens applied.

### Background Art

The number of contact lens wearers has continued to increase over the past thirty years. Contact lenses, which are a type of intraocular lenses, are broadly classified into soft contact lenses and hard contact lenses. The soft contact lens is softer and more comfortable to wear than the hard contact lens, so that the soft contact lens has been widely spread. However, even when the soft contact lens is worn, friction can occur in the blinking of eye between the surface of a contact lens and the ocular tissue, such as between the periphery portion of the soft contact lens and the bulbar conjunctiva, between the inner surface of the soft contact lens and the outer membrane of eyeball, and between the outer surface of the soft contact lens and the eyelid conjunctiva. The friction can lead to the breakdown of tear fluid layer and the development of ocular disorders.

Furthermore, in recent years, it is believed that the friction caused between the surface of the contact lens worn and the ocular tissue correlates with the comfort in wearing the contact lens. Since the lower friction provides the improved comfort, attempts have been made to reduce the friction that occurs between the surface of the contact lens worn and the ocular tissue.

Examples of such attempts include a silicone hydrogel with a friction coefficient reduced by overnight washing with a package solution containing alkylaryl-containing surfactant with an ophthalmologically acceptable concentration in the range between about 0.01 wt% and 0.5 wt% (see, e.g., Patent Document 1 below, the disclosure of which is incorporated herein by reference in its entirety).

### Citation List

### Patent Documents

Patent Document 1: JP 2019-509516 A

### Summary of the Invention

### Problems to be Solved by the Invention

However, the present inventors have found that the single use of surfactant, as described in Patent Document 1, has a smaller friction-reducing effect, and furthermore, such a friction-reducing effect is instantaneous but not durable. In fact, in Patent Document 1, the friction coefficient has been measured only once, immediately after treatment with the package solution, and the durability of the treatment effect has not been evaluated.

Accordingly, it is an objective of the present invention to provide a contact lens solution that can continuously reduce friction on the surface of a contact lens, such as hydrogel contact lens and that can be used to obtain a contact lens having surface smoothness.

### Means of Solving the Problems

In order to solve the above-identified problems, the present inventors earnestly studied over hundreds of components used in contact lens solutions. As a result, the present inventors found out that some components could solely reduce friction on the surface of a contact lens, but that their friction-reducing effects were not durable. Through further trial-and-error processes, the present inventors attempted to obtain a contact lens solution that can have an excellent friction-reducing effect on the surface of a contact lens but such an effect is durable.

Surprisingly, it was found out that treating the contact lens with a solution containing a nonionic surfactant and a polysaccharide allowed the coefficient of friction on the surface of the contact lens to be reduced and further allowed the reduced coefficient to be durable. Finally, based on such findings, the present inventors have succeeded in inventing a contact lens solution containing a nonionic surfactant and a polysaccharide as a possible solution to the problems. As such, the present invention has been completed on the basis of the findings and successful examples.

According to the present invention, there is provided a contact lens solution and a method in the following aspects:
[1] a contact lens solution comprising a nonionic surfactant and a polysaccharide.
[2] the contact lens solution according to [1], further comprising at least one component selected from the group consisting of a buffering agent, an isotonicity agent and a chelating agent.
[3] the contact lens solution according to [1] or [2], wherein the contact lens solution is a contact lens solution for smoothing the surface of a contact lens.
[4] the contact lens solution according to any one of [1] to [3], wherein the nonionic surfactant is at least one nonionic surfactant selected from the group consisting of polyoxyethylene-based surfactants, and/or the polysaccharide is at least one polysaccharide selected from the group consisting of mucopolysaccharides and cellulose-based polymer compounds.
[5] the contact lens solution according to any one of [1] to [4], wherein the mass ratio of the nonionic surfactant and the polysaccharide (nonionic surfactant:polysaccharide) is 1:0.01 to 1:50 and/or the total content of the nonionic surfactant and the polysaccharide is 0.001 w/v% to 5.0 w/v% relative to the total amount of the solution.
[6] the contact lens solution according to any one of [2] to [5], wherein the ratio ([B]:[A]) of the total content ([B]) of the buffering agent, the isotonicity agent and the chelating agent relative to the total content ([A]) of the nonionic surfactant and the polysaccharide is 1:0.1 to 1:100.
[7] the contact lens solution according to any one of [1] to [6], wherein the contact lens solution is a contact lens package solution or a contact lens care solution.
[8] a method for smoothing the surface of a contact lens, comprising bringing a contact lens into contact with the contact lens solution according to any one of [1] to [7].
[9] a method of producing a contact lens having surface smoothness, comprising bringing a contact lens into contact with the contact lens solution according to any one of [1] to [7] to obtain the contact lens having surface smoothness.

### Effects of the Invention

The contact lens solution according to one embodiment of the present invention can durably reduce the friction coefficient on the surface of a contact lens, thereby smoothing the surface of the contact lens. Therefore, the contact lens treated with the contact lens solution according to one embodiment of the present invention is expected to reduce friction between the surface of the contact lens and the ocular tissue and provide an excellent wearing comfortableness.

It is expected that the method according to one embodiment of the present invention can durably reduce the friction coefficient on the surface of a contact lens and can smooth the surface of the contact lens, thereby continuously suppressing the breakdown of tear fluid layer and the development of ocular disorders caused by the friction occurring while wearing and blinking.

### Brief Description of the Figures

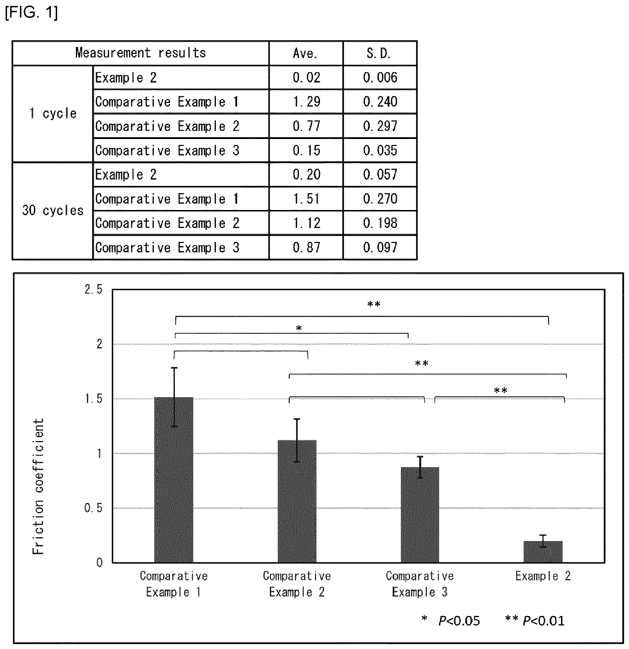

[FIG. 1] FIG. 1 is a table summarizing the results of the friction coefficient measured using the contact lens solutions of Example 2 and Comparative Examples 1 to 3 and a graph demonstrating the results of the average friction coefficient after 30 cycles of treatment, as described in Examples below. Symbols in the graph indicate the results of t-tests between each test group, with "*" indicating a significant difference at the 5% significance level and "**" indicating a significant difference at the 1% significance level.

### Modes for Carrying Out the Invention

While each aspect that forms one embodiment of the present invention will now be described in detail, the present invention is not limited only by the matters of this section, and may take various forms to the extent that its objective can be achieved.

Unless otherwise specified, each term used herein is used in the meaning commonly used by those skilled in the art, in particular in the ocular lens field, and should not be construed to have any meaning that is unduly limiting. Also, any speculations and theories herein are made on the basis of the knowledge and experiences of the present inventors and as such, the present invention is not bound by any such speculations and theories.

The term "content" is synonymous with concentration, and means the ratio of the amount of a component relative to the total amount of the solution. However, the total content of the components may not exceed 100%.

The term "and/or" as used herein means either any one of, any combination of two or more of, or combination of all of listed related items.

The wording "to" for indicating a range of values is intended to include values preceding and following the wording; for example, "0% to 100%" means a range from 0% or more and 100% or less. The terms "more than" and "less than" used herein means the lower and upper limits without including a value following the term, respectively. For example, "more than 1" means a value beyond 1, and "less than 100" means a value below 100.

The terms "include," "comprise," and "contain" mean that an element(s) other than an element(s) as explicitly indicated can be added as inclusions, which are, for example, synonymous with "at least include," but encompasses the meaning of "consist of" and "substantially consist of". In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

The terms "friction-reducing effect" used herein means the effect in reducing the average friction coefficient by bring a contact lens into contact with a contact lens solution, when the average friction coefficient on the surface of the contact lens is measured according to the method described in Examples below.

The terms "durably friction-reducing effect" means the effect in confirming the friction-reducing effect, and further suppressing or mitigating the increase in the average friction coefficient after 30 cycles of treatment as compared to the average friction coefficient after 1 cycle of treatment, when the average friction coefficient on the surface of a contact lens is measured according to the method described in Examples below.

The terms "surface smoothness of contact lens" means the ability that by bringing a contact lens into contact with a contact lens solution, the friction-reducing effect and/or the durably friction-reducing effect can be exerted so as to suppress or mitigate the occurrence of friction between the contact lens and the ocular tissue, thereby smoothing the surface of the contact lens.

The contact lens solution according to one embodiment of the present invention is characterized by containing a nonionic surfactant and a polysaccharide.

One aspect of the method of the present invention is a method for smoothing the surface of a contact lens, including bringing a contact lens, preferably a contact lens composed of hydrogel, into contact with the contact lens solution according to one embodiment of the present invention. Another aspect of the method of the present invention is a method of producing a contact lens having surface smoothness, including bringing a contact lens into contact with the contact lens solution according to one embodiment of the present invention to obtain the contact lens having surface smoothness. When the contact lens obtained by the method according to one embodiment of the present invention is worn, it is expected to durably reduce various types of inflammation and unpleasant sensation caused by friction occurring between the contact lens and the ocular tissue, such as between the contact lens and the ocular surface and between the contact lens and the eyelid conjunctiva.

The nonionic surfactant is not particularly limited as long as the nonionic surfactant is one normally known. Examples of the nonionic surfactant include ether type, ester ether type, ester type and alkanolamide type nonionic surfactants. From the viewpoint of imparting desirable wettability to the contact lens, the nonionic surfactant is preferably selected from ether type nonionic surfactants, and more preferably selected from polyoxyethylene-based surfactants among ether type nonionic surfactants.

On the other hand, some of polyoxyethylene-based surfactants are compounds that are toxic to ocular tissues. There is a risk of ocular disorders when a subject wears a contact lens which has adsorbed compounds that are toxic to ocular tissues. Therefore, when selecting a polyoxyethylene-based surfactant as the nonionic surfactant, the following surfactants may be preferably used: polyoxyethylene polyoxypropylene glycol obtained by addition polymerization of polypropylene glycol with ethylene oxide; tetrafunctional block copolymer obtained by copolymerizing copolymer composed of polyoxyethylene and polyoxypropylene with ethylenediamine; and polyoxyethylene hardened castor oil obtained by etherifying hardened castor oil with polyoxyethylene chain.

In polyoxyethylene polyoxypropylene glycol, and tetrafunctional block copolymer composed of polyoxyethylene polyoxypropylene copolymer and ethylenediamine, the content of ethylene oxide in the total molecular as a molecular structure is preferably 40% to 95%, and/or the ratio of the mole number of propylene oxide added relative to the mole number of ethylene oxide added is preferably 1:0.1 to 1:0.85, in view of the solubility in water and the relationship between the content of ethylene oxide and the cloud point. For example, using one having the excessively lower content of ethylene oxide may cause cloudiness due to a lower cloud point.

Examples of polyoxyethylene polyoxypropylene glycol include polyoxyethylene (50) polyoxypropylene (40) glycol, polyoxyethylene (300) polyoxypropylene (55) glycol, polyoxyethylene (200) polyoxypropylene (40) glycol, polyoxyethylene (160) polyoxypropylene (30) glycol, polyoxyethylene (196) polyoxypropylene (67) glycol, and polyoxyethylene (200) polyoxypropylene (70) glycol. As polyoxyethylene polyoxypropylene glycol, commercially available products such as Kolliphor P188 and Kolliphor P407 (manufactured by BASF, respectively), and Pluronic (registered trademark) L-64, P-84, P-85, F-68, F-88, F-108, F-77 and F-127 (manufactured by ADEKA, respectively) can be used.

Examples of tetrafunctional block copolymer composed of polyoxyethylene polyoxypropylene copolymer and ethylenediamine include ethylenediamine tetrapolyoxyethylene polyoxypropylene. As tetrafunctional block copolymer composed of polyoxyethylene polyoxypropylene copolymer and ethylenediamine, commercially available products such as Tetoronic 904, 1104, 1107, 1304, 1307, and 1504 (manufactured by BASF, respectively), and Pluronic (registered trademark) TR-704 and TR-913R (manufactured by ADEKA, respectively) can be used.

In polyoxyethylene hardened castor oil, the mole number of ethylene oxide added as a molecular structure is preferably 40 to 60, and/or Hydrophile-Lipophile Balance value (degree of affinity to water and oil for surfactant: HLB value) as a physicochemical property is preferably 12.5 to 16.

Examples of polyoxyethylene hardened castor oil include polyoxyethylene hardened castor oil 40 and polyoxyethylene hardened castor oil 60. As polyoxyethylene hardened castor oil, commercially available products such as Kolliphor RH-40 (manufactured by BASF), HCO-4 0, HCO-50 and HCO-60 (manufactured by Nikko Chemical, respectively) can be used.

As other polyoxyethylene-based surfactants, compounds listed in the official specifications of various countries including the Japanese Pharmacopoeia, the Japanese Standards of Quasi-Drug Ingredients 2006 (JSQI), the United States Pharmacopoeia and the European Pharmacopoeia may be employed.

The nonionic surfactant may be used either individually or in combination of two or more of the above nonionic surfactants.

In the contact lens solution according to one embodiment of the present invention, the content of the nonionic surfactant may be appropriately set according to the type and structure of the compound employed as the nonionic surfactant and is not particularly limited, as long as the content allows the contact lens applied to have surface smoothness. For example, in view of the effect on safety to ocular tissues, the content is preferably in the range between 0.0001 w/v% and 5.0 w/v% and more preferably in the range between 0.0005 w/v% and 2.5 w/v%, and from the viewpoint of continual friction reduction, still more preferably in the range between 0.001 w/v% and 1 w/v%.

The polysaccharide has a polymeric structure in which a number of monosaccharide molecules of single or different structures are bound to each other via glycosidic bonds. Examples of the polysaccharide include, but are not particularly limited to, mucopolysaccharides, xanthan gum, triglucopolysaccharides and cellulose-based compounds. The polysaccharide may also take the form of salts such as sodium salts, potassium salts and calcium salts.

The polysaccharide is preferably selected from the group consisting of mucopolysaccharides, triglucopolysaccharides and cellulose-based polymer compounds from the viewpoint of safety.

While the mucopolysaccharide may have a structure in which a hydroxyl group of a sugar molecule is substituted by an amino group, examples of the mucopolysaccharide include hyaluronic acid, chondroitin sulfate, chitosan and alginic acid.

While the triglucopolysaccharide may have a structure in which three glucose molecules form a single unit and several units are bound to each other, examples of the triglucopolysaccharide include Pullulan PI-20 and Pullulan PF-20 (manufactured by Hayashibara, respectively).

The cellulose-based compound may have a structure in which a hydroxyl group of cellulose, which has a polymeric structure in which many β-glucoses are bound to each other in the linear manner, is substituted by another functional group. Examples of the functional group include methoxy group, ethoxy group, hydroxymethoxy group, hydroxyethoxy group, hydroxypropoxy group, carboxymethoxy group and carboxyethoxy group. Examples of the cellulose-based compound include methyl cellulose, ethyl cellulose, hydroxyethyl cellulose, hydroxymethyl cellulose, hydroxypropyl cellulose, hydroxypropylmethyl cellulose, carboxylmethyl cellulose and carboxylethyl cellulose.

While the preferred examples of the polysaccharide include hyaluronic acid, alginic acid, chondroitin sulfate, hydroxypropylmethyl cellulose, hydroxyethyl cellulose and carboxymethyl cellulose, the polysaccharide is more preferably alginic acid.

The polysaccharide may be used either individually or in combination of two or more of the above polysaccharides.

In the contact lens solution according to one embodiment of the present invention, the content of the polysaccharide may be appropriately set according to the type and structure of the compound employed as the polysaccharide as well as the application form and is not particularly limited, as long as the content allows the contact lens applied to have surface smoothness. For example, the content is preferably in the range between 0.0001 w/v% and 1 w/v% and more preferably in the range between 0.0005 w/v% and 0.75 w/v%, and from the viewpoint of continual friction reduction, still more preferably in the range between 0.001 w/v% and 0.5 w/v%. When the contact lens solution according to one embodiment of the present invention having the content of the polysaccharide of more than 1 w/v% is subjected to high-pressure steam sterilization, the entire solution may turn yellow depending on the type of polysaccharide, such as alginic acid.

In the contact lens solution according to one embodiment of the present invention, a certain ratio of the nonionic surfactant content and the polysaccharide content can impart the desired surface smoothness to the contact lens applied. For this purpose, the mass ratio of the nonionic surfactant and the polysaccharide (nonionic surfactant:polysaccharide) may be preferably 1:0.001 to 1:100, more preferably 1:0.005 to 1:75, and still more preferably 1:0.01 to 1:50.

When the total content of the nonionic surfactant and the polysaccharide is an effective amount, the contact lens applied can have surface smoothness. Therefore, the total content of the nonionic surfactant and the polysaccharide may be, relative to the total amount of the solution, preferably 0.001 w/v% to 5.0 w/v%, more preferably 0.005 w/v% to 2.5 w/v%, and still more preferably 0.01 w/v% to 1.5 w/v%. If the total content of the nonionic surfactant and the polysaccharide is less than 0.001 w/v%, the solution tends to impart no surface smoothness to the contact lens applied and thus the total content is undesirable.

In addition to the nonionic surfactant and the polysaccharide, the contact lens solution according to one embodiment of the present invention preferably contains at least one or two of, or all three of a buffering agent, an isotonicity agent and a chelating agent in view of the shape of contact lens and the irritation to the eye.

Each of the buffering agent, the isotonicity agent and the chelating agent is not particularly limited, as long as it is a compound normally employed in contact lens solutions and ophthalmic solvents.

Examples of the buffering agent include phosphate buffers, borate buffers, citrate buffers, amino acid buffers and tris (trishydroxyaminomethane) buffers. Among the examples, the buffering agent is preferably borate buffers and phosphate buffers, which are commonly employed in contact lens solutions, and more preferably borate buffers. The content of the buffering agent is not particularly limited, as long as the buffering effect is exerted. Examples of the content include such amounts that the contact lens solution has a pH value of preferably near neutral, more preferably 6.0 to 9.0, and still more preferably 6.8 to 8.0.

Examples of the isotonicity agent include glycerin, propylene glycol, propanediol, mannitol, sorbitol, sodium carbonate, sodium bicarbonate, sodium chloride, and potassium chloride. Among the examples, the isotonicity agent is preferably sodium chloride, potassium chloride, propylene glycol and propanediol, which are commonly employed in contact lens solutions. The content of the isotonicity agent is not particularly limited, as long as the osmotic pressure of the contact lens solution becomes the similar or same as the osmotic pressure of the ocular tissue. Examples of the content include such amounts that the osmotic pressure of the contact lens solution becomes preferably in the range between 200 mmol/kg and 400 mmol/kg.

Examples of the chelating agent include polyvalent carboxylic acids and their salts such as edetic acid (ethylenediaminetetraacetic acid), sodium edetate, disodium edetate, citric acid and tartaric acid. Among the examples, the chelating agent is preferably ethylenediaminetetraacetic acid and citric acid, which are commonly employed in contact lens solutions. The content of the chelating agent is not particularly limited, as long as it is in an amount sufficient to capture metal ions in the contact lens solution. Examples of the content include preferably 0.001 w/v% to 1.0 w/v% and more preferably 0.01 w/v% to 0.1 w/v%.

For the purpose of ensuring that the contact lens solution according to one embodiment of the present invention contains the combination of the nonionic surfactant and the polysaccharide thereby imparting surface smoothness to the contact lens applied, it is preferable that the total content of the buffering agent, the isotonicity agent and the chelating agent is not too excessive with respect to the total content of the nonionic surfactant and the polysaccharide. From this standpoint, the ratio ([B]/[A]) of the total content ([B]) of the buffering agent, the isotonicity agent and the chelating agent contained in the contact lens solution relative to the total content ([A]) of the nonionic surfactant and the polysaccharide contained in the contact lens solution is preferably 1:0.1 to 1:100, and 1:1 to 1:20.

When the contact lens solution according to one embodiment of the present invention is subjected to heat treatment including high-pressure steam sterilization, some of the components contained may decompose. Therefore, the above range with respect to each component content is preferably the amount before heat treatment. Even if a value below the above range is obtained when the content of each component is measured for the solution after heat treatment, the content of each component may be considered to be within the above range by analogous application of a value before heat treatment.

If the nonionic surfactant and the polysaccharide are contained at an effective amount and further the total content of the buffering agent, the isotonicity agent and the chelating agent is set within a predetermined range, the contact lens solution according to one embodiment of the present invention can develop positive effects on the shape of the contact lens applied and the irritating sensation to the eye, while reducing friction on the contact lens surface and imparting surface smoothness to the contact lens applied.

The contact lens solution according to one embodiment of the present invention may further contain other components in addition to the nonionic surfactant, the polysaccharide, the buffering agent, the isotonicity agent and the chelating agent, as long as it does not prevent such other components from solving the problems of the present invention. While the other component is not particularly limited, examples of the other component include those normally contained in ophthalmic solutions such as contact lens solutions and eye drops.

The solvent used to prepare the contact lens solution according to one embodiment of the present invention is preferably water. The content of water is preferably about 96 parts to 99 parts relative to total 100 parts of the solution.

Since the contact lens solution according to one embodiment of the present invention is used for contact lenses, the contact lens solution preferably has properties that do not develop negative effects on contact lenses and/or ocular tissues.

For example, the pH value of the contact lens solution is preferably near neutral, more preferably 6.0 to 9.0, still more preferably 6.8 to 8.0 and even still more preferably 7.0 to 7.6. If the pH value does not fall within the above range, it may be appropriately adjusted using an acid such as hydrochloric acid or an alkali such as sodium hydroxide. The osmotic pressure of the contact lens solution is preferably 200 mmol/kg to 400 mmol/kg and more preferably 250 mmol/kg to 350 mmol/kg. The viscosity of the contact lens solution is preferably 0.1 mPa.s to 10 mPa.s, more preferably 0.5 mPa.s to 5 mPa.s, and still more preferably 0.8 mPa.s to 2 mPa.s. The coloration of the contact lens solution is preferably clear, and more preferably colorless and transparent. The pH value, osmotic pressure, viscosity and coloration of the contact lens solution are measured according to the methods described in Examples below, respectively.

A non-limiting preferred embodiment of the contact lens solution of the present invention is a contact lens solution containing:
(1) nonionic surfactant: 0.01 w/v% to 0.5 w/v%; and
(2) polysaccharide: 0.01 w/v% to 0.5 w/v%;
wherein Component (1):Component (2) = 1:0.02 to 1:50, and Component (1) + Component (2) = 0.02 w/v% to 1.0 w/v%.

A non-limiting preferred embodiment of the contact lens solution of the present invention is a contact lens solution containing:
(1) nonionic surfactant: 0.01 w/v% to 0.5 w/v%;
(2) polysaccharide: 0.01 w/v% to 0.5 w/v%; and
(3) buffering agent, isotonicity agent and chelating agent: 0.5 w/v% to 5.0 w/v%;
wherein Component (1):Component (2) = 1:0.02 to 1:50, Component (1) + Component (2) = 0.02 w/v% to 1.0 w/v%, and Component (3):[Component (1) + Component (2)] = 1:0.1 to 1:50.

The contact lens solution according to one embodiment of the present invention can be produced by a combination of steps known by those skilled in the art, and the production method is not particularly limited, but can include, for example, the following steps of:
adding and mixing a nonionic surfactant and a polysaccharide, and optionally, a buffering agent, an isotonic agent, a chelating agent and other additives to purified water to obtain a mixed solution; stirring the obtained mixed solution to obtain a uniform solution; filtering the obtained uniform solution to obtain a filtrate solution; and filling the obtained filtrate solution into a predetermined container to obtain a contact lens solution.

The contact lens solution is preferably subjected to heat sterilization either in the solution form before filling or in the whole container after filling.

While the usage method of the contact lens solution according to one embodiment of the present invention is not particularly limited, it may be used so as to bring the contact lens solution in contact with a contact lens. The detailed mechanism by which the contact lens solution according to one embodiment of the present invention allows the contact lens applied to have surface smoothness is not clearly known, but it may rely on the fact that the contact lens solution according to one embodiment of the present invention coats the surface of the contact lens applied. Accordingly, the contact lens solution according to one embodiment of the present invention is preferably used in such a manner that the solution adheres to the entire surface of the contact lens applied.

Examples of the preferred modes of the contact lens solution according to one embodiment of the present invention include a solution for packing a contact lens which is filled into a container together with a contact lens when shipping the contact lens; a solution for disinfecting, washing or preserving a contact lens, or a solution for caring a contact lens which is used in order to disinfect, wash and preserve the contact lens; a solution for wearing a contact lens and an eye drop formulation that are used in wearing the contact lens. Examples of more preferred modes of the contact lens solution according to one embodiment of the present invention include the solution for packing a contact lens and the solution for caring a contact lens because they can be brought in contact with the entire contact lens for an extended period of time.

The present invention will now be described in further detail with reference to Examples, which are not intended to limit the present invention. The present invention may take various embodiments to the extent that the objectives of the present invention are achieved.

### Examples

### [1. Preparation and characterization of contact lens solution]

According to the formulations as shown in Tables 1 to 3, the polyoxyethylene-based surfactants, polysaccharides, buffering agents, isotonicity agents and chelating agents were added, and the mixture was made up to 1 L using purified water. The obtained mixture was stirred at room temperature (20°C to 25°C) for 120 minutes, and then filtered through a membrane filter to obtain the contact lens solutions of Examples 1 to 21, Comparative Examples 1 to 13 and Reference Examples 1 to 2.

The pH value, osmotic pressure, viscosity and coloration of the solution were measured for the obtained contact lens solutions.

The pH value was measured at room temperature using a pH meter ("Main Unit: Seven Compact S220, Electrode: InLab MicroPro-ISM ISM"; manufactured by Mettler Toledo).

The osmotic pressure was measured at room temperature using an osmometer ("Model 3250"; manufactured by ADVANCE INSTRUMENTS).

The viscosity was measured according to the spindle-rotor method using the RB80 type viscometer (manufactured by Toki Sangyo) in such a manner that 20 mL of sample was adjusted at 20°C, in which BL adapter (Code No. 10) was immersed, and the sample was then measured at 60 rpm for 3 minutes.

The coloration of solution was evaluated by visual observation, with "None" for transparency and "Yes" for yellowish or other coloration.

### [2. Preparation of hydrogel contact lens]

### (2-1) Hydrogel contact lens 1

The monomer mixture obtained by mixing 95 g of 2-hydroxyethyl methacrylate, 4.5 g of methacrylic acid and 0.5 g of ethylene glycol dimethacrylate was stirred at room temperature until uniform. To the mixture, 0.3 g of 2,2-azobisisobutyronitrile was added, and then the obtained mixture was further stirred.

The resulting pre-reaction solution was injected into a polypropylene contact lens mold and heated from room temperature to 100°C under a nitrogen atmosphere to obtain a contact lens-shaped molded body. The resulting contact lens-shaped molded body was immersed and swelled in a phosphate buffer solution warmed to 70°C for 30 minutes to obtain hydrogel contact lens 1.

### (2-2) Hydrogel contact lens 2

The commercially available hydrogel lens "1-DAY Acuvue" (manufactured by Johnson & Johnson) was taken from the packaging container and then subjected to immersion treatment with the phosphate buffer solution at room temperature. The immersion treatment was performed at room temperature for 3 days while changing the phosphate buffer solution every 12 hours. The contact lens after the immersion treatment was designated as hydrogel contact lens 2.

### [3. Treatment of hydrogel contact lens with contact lens solution]

After dispensing each contact lens solution into an empty blister container in the amount of about 80% of the container volume, hydrogel contact lens 1 or hydrogel contact lens 2 was immersed into the container, and then the opening of the blister container was sealed with a sealant. The sealed blister container was subjected to high-pressure steam sterilization at 121°C for 20 minutes to treat the hydrogel contact lens with the contact lens solution.

### [4. Evaluation of friction on contact lens surface using the contact lens solutions listed in Tables 1 and 2]

### (4-1) Measurement method of friction coefficient

The evaluation of friction on the surface of the contact lens was performed based on the friction coefficient of the contact lens surface measured according to the following method using a friction coefficient analyzer ("NanoTribometer TIX-NTR3"; manufactured by Anton Paar).

The hydrogel contact lens treated with the contact lens solution was taken from the packaging container, the surface moisture was removed, and then the contact lens was mounted on the friction measurement fixture. About 6.5 mL of PBS (-) was dropped onto the hydrogel contact lens to moisten the surface of the contact lens. The friction coefficient was measured for the moistened hydrogel contact lens by using the friction coefficient analyzer, which was adjusted such that the center of the probe of the analyzer was aligned with the center of the contact lens, according to the following analytical condition (measurement after 1 cycle of treatment). The measurement of this analysis condition was repeated 30 times and the friction coefficient after 30 measurements was recorded (measurement after 30 cycles of treatment).

### [Analytical condition of friction coefficient analyzer]

Substrate: Sapphire Ball 3.0 mm
Approach Speed: 5.0 µm/s
Full amplitude: 1.0 mm
Max linear speed: 0.1 mm/s
Trajectory: Sinus
Sequence count: 1
Load: 2.0 mN
Cycles: 30 to 60

### (4-2) Friction evaluation method

Three to five blister containers were prepared for each contact lens solution. Hydrogel contact lens 1 was used as the contact lens applied. The friction coefficient was measured for each hydrogel contact lens 1 treated with the contact lens solution once per one lens, and the average value of the friction coefficients measured was calculated. The average value of the friction coefficients obtained from the measurement after 1 cycle of treatment was used to evaluate the surface smoothness of the contact lens according to the following criteria.

Furthermore, the average friction coefficient obtained from the measurement after 30 cycles of treatment and the average friction coefficient obtained from the measurement after 1 cycle of treatment were used to evaluate the durability of the surface smoothness of the contact lens according to the following criteria.

### [Evaluation criteria 1 for surface smoothness of contact lens]

++: Average friction coefficient of less than 0.10
+: Average friction coefficient of between 0.10 or more and less than 0.30
-: Average friction coefficient of 0.30 or more

### [Evaluation criteria 1 for durability of surface smoothness of contact lens]

++: Difference between the average friction coefficient after 30 cycles of treatment and the average friction coefficient after 1 cycle of treatment of less than 0.30
+: Difference between the average friction coefficient after 30 cycles of treatment and the average friction coefficient after 1 cycle of treatment of between 0.30 or more and less than 0.50
-: Difference between the average friction coefficient after 30 cycles of treatment and the average friction coefficient after 1 cycle of treatment of 0.50 or more

### (4-3) Evaluation results: Examples 1 to 7 and Comparative Examples 1 to 3

Table 1A shows the pH value, osmotic pressure, viscosity and coloration of each contact lens solution, as well as the average friction coefficient and friction evaluation result of the surface of the contact lens treated with each contact lens solution.

As shown in Table 1A, the results obtained using the contact lens solutions of Examples 1 to 7 demonstrated that the contact lens treated with the contact lens solution containing the polyoxyethylene-based surfactant P407 (polyoxyethylene (196) polyoxypropylene (67) glycol) and the polysaccharide alginic acid could have a smaller average friction coefficient after 1 cycle of treatment and after 30 cycles of treatment, and have a durably excellent surface smoothness.

In addition, FIG. 1 shows a summary of the measurement results of the friction coefficient as well as a graph of the measurement results of the average friction coefficient after 30 cycles of treatment, when using each contact lens solution of Example 2 and Comparative Examples 1 to 3. In FIG. 1, the results of t-tests between test groups are shown together.

As shown in FIG. 1, the contact lens solution of Comparative Example 2 containing only alginic acid was not significantly different from the contact lens solution of Comparative Example 1 containing neither alginic acid nor P407. In contrast, the contact lens solution of Example 2 containing the combination of alginic acid and P407 was significantly different at the 1% significance level from Comparative Example 1.

Furthermore, the contact lens solution of Comparative Example 3 containing only P407 was not significantly different from the contact lens solution of Comparative Example 2 containing only alginic acid. In contrast, the contact lens solution of Example 2 containing both alginic acid and P407 was significantly different at the 1% significance level from not only the contact lens solution of Comparative Example 2 but also from the contact lens solution of Comparative Example 3 containing only P407.

Taken together, it can be concluded that the durably excellent friction-reducing effect on the surface of the contact lens achieved by the contact lens solution containing the combination of alginic acid and P407 is not an additive effect by combining the individual components but a synergistic effect by combining the individual components.

On the other hand, for the contact lens solutions of Example 2 and Comparative Examples 1 to 3, Table 1B shows the average friction coefficient and friction evaluation results for the surface of the contact lens under the condition that the sealed blister container was not subjected to high-pressure steam sterilization treatment in the treatment of the hydrogel contact lens with the contact lens solution.

From the results shown in Table 1B, it was found out that, with or without high-pressure steam sterilization treatment, the contact lens solution containing the combination of alginic acid and P407 had a durably excellent friction-reducing effect on the surface of a contact lens.

### (4-4) Evaluation results: Examples 8 to 18 and Comparative Examples 4 to 7

Table 2 shows the pH value, osmotic pressure and coloration of each contact lens solution, as well as the average friction coefficient and friction evaluation results for the surface of the contact lens treated with each contact lens solution.

As shown in Table 2, the results of the contact lens solutions of Examples 8 to 18 demonstrated that the contact lens treated with the contact lens solution containing a combination of a polyoxyethylene-based surfactant and a polysaccharide could have a smaller average friction coefficient after 1 cycle of treatment and after 30 cycles of treatment, and could have a durably excellent surface smoothness, even if the contact lens solution contained a variety of polyoxyethylene-based surfactants, polysaccharides, buffering agents, isotonicity agents and chelating agents.

To the contrast, it was found out that the contact lens treated with each contact lens solution of Comparative Examples 4 to 7 containing no polyoxyethylene-based surfactant and/or no polysaccharide had no effect in reducing the average friction coefficient after 1 cycle of treatment, or rather showed an increase in the average friction coefficient after 30 cycles of treatment.

These results indicate that the contact lens solution containing a polyoxyethylene-based surfactant and a polysaccharide has an excellent and durably friction-reducing effect on the surface of a contact lens.

### [5. Friction evaluation of contact lens surface using the contact lens solutions listed in Table 3]

### (5-1) Friction evaluation method

The friction coefficients were measured after 1 cycle of treatment and after 30 cycles of treatment and the average friction coefficient was calculated for the contact lens treated with each contact lens solution listed in Table 3 in the same manner as in the section (4-2) above, except that hydrogel contact lens 2 was employed as the contact lens. The average friction coefficient after 1 cycle of treatment was used to evaluate the surface smoothness of contact lens according to the following criteria. Furthermore, the average friction coefficient after 30 cycles of treatment and the average friction coefficient after 1 cycle of treatment were used to evaluate the durability of the surface smoothness of contact lens according to the following criteria.

### [Evaluation criteria 2 for surface smoothness of contact lens]

++: Average friction coefficient of less than 0.10
+: Average friction coefficient of between 0.10 or more and less than 0.30
-: Average friction coefficient of 0.30 or more

### [Evaluation criteria 2 for durability of surface smoothness of contact lens]

++: Difference between the average friction coefficient after 1 cycles of treatment and the average friction coefficient after 30 cycles of treatment of less than 0.15
+: Difference between the average friction coefficient after 1 cycles of treatment and the average friction coefficient after 30 cycles of treatment of between 0.15 or more and less than 0.30
-: Difference between the average friction coefficient after 1 cycles of treatment and the average friction coefficient after 30 cycles of treatment of 0.30 or more

### (5-2) Evaluation results: Examples 20 and 21, and Comparative Examples 9 to 13

Table 3 shows the average friction coefficient on the surface of the contact lens treated with each contact lens solution and the friction evaluation result.

As shown in Table 3, it was found out that the contact lens solution containing a polyoxyethylene-based surfactant and a polysaccharide had an excellent and durably friction-reducing effect on the surface of the contact lens even when the type of contact lens was changed; and the contact lens treated with the contact lens solution containing no polyoxyethylene surfactant and/or no polysaccharide had no effect in reducing the average friction coefficient after 1 cycle of treatment or showed an increase in the average friction coefficient after 30 cycles of treatment.

**[Table 1A]**

| | | Examples | | | | | | | Comparative Examples | | |
|---|---|---|---|---|---|---|---|---|---|---|---|
| | | 1 | 2 | 3 | 4 | 5 | 6 | 7 | 1 | 2 | 3 |
| Surfactant (w/v%) | P407 | 0.01 | 0.05 | 0.1 | 0.1 | 0.01 | 0.5 | 1 | - | - | 0.05 |
| Polysaccharide (w/v%) | Alginic acid | 0.01 | 0.1 | 0.1 | 0.05 | 0.5 | 0.01 | 0.1 | - | 0.1 | - |
| Buffering agent (w/v%) | Boric acid | 0.99 | 0.99 | 0.99 | 0.99 | 0.99 | 0.99 | 0.99 | 0.99 | 0.99 | 0.99 |
| | Borax | 0.38 | 0.38 | 0.38 | 0.38 | 0.38 | 0.38 | 0.38 | 0.38 | 0.38 | 0.38 |
| Isotonicity agent (w/v%) | Sodium chloride | 0.3 | 0.3 | 0.3 | 0.3 | 0.3 | 0.3 | 0.3 | 0.3 | 0.3 | 0.3 |
| Chelating agent (w/v%) | Ethylenediaminetetraacetic acid | 0.05 | 0.05 | 0.05 | 0.05 | 0.05 | 0.05 | 0.05 | 0.05 | 0.05 | 0.05 |
| 1N HCl (µL) | | 0.65 | - | - | 0.4 | - | 0.65 | - | 0.65 | - | 0.65 |
| 1N NaOH (µL) | | - | - | - | - | 1.4 | - | - | - | - | - |
| Purified water | | Bal. | Bal. | Bal. | Bal. | Bal. | Bal. | Bal. | Bal. | Bal. | Bal. |
| Total amount | | 100 ml | 100 ml | 100 ml | 100 ml | 100 ml | 100 ml | 100 ml | 100 ml | 100 ml | 100 ml |
| pH | | 7.45 | 7.46 | 7.45 | 7.45 | 7.30 | 7.45 | 7.46 | 7.45 | 7.45 | 7.48 |
| Osmotic pressure (mOsm) | | 307 | 293 | 295 | 301 | 309 | 307 | 308 | 305 | 293 | 305 |
| Viscosity (mPa·s) | | 1.11 | 1.16 | 1.17 | 1.13 | 1.34 | 1.21 | - | 1.11 | 1.14 | 1.11 |
| Coloration of solution | | None | None | None | None | None | None | None | None | None | None |
| Hydrogel contact lens applied | | 1 | 1 | 1 | 1 | 1 | 1 | 1 | 1 | 1 | 1 |
| Friction coefficient (Ave.) | 1 | 0.11 | 0.02 | 0.01 | 0.01 | 0.05 | 0.12 | 0.07 | 1.29 | 0.77 | 0.15 |
| | 30 | 0.29 | 0.20 | 0.07 | 0.04 | 0.30 | 0.27 | 0.22 | 1.51 | 1.12 | 0.87 |
| Surface smoothness | | + | ++ | ++ | ++ | ++ | + | ++ | - | - | + |
| Durability of surface smoothness | | ++ | ++ | ++ | ++ | ++ | ++ | ++ | | | |

**[Table 1B]**

| | | Example | Comparative Examples | | |
|---|---|---|---|---|---|
| | | 2 | 1 | 2 | 3 |
| Surfactant (w/v%) | P407 | 0.05 | - | - | 0.05 |
| Polysaccharide (w/v%) | Alginic acid | 0.1 | - | 0.1 | - |
| Buffering agent (w/v%) | Boric acid | 0.99 | 0.99 | 0.99 | 0.99 |
| | Borax | 0.38 | 0.38 | 0.38 | 0.38 |
| Isotonicity agent (w/v%) | Sodium chloride | 0.3 | 0.3 | 0.3 | 0.3 |
| Chelating agent (w/v%) | Ethylenediaminetetraacetic acid | 0.05 | 0.05 | 0.05 | 0.05 |
| 1N HCl (µL) | | - | 0.65 | - | 0.65 |
| 1N NaOH (µL) | | - | - | - | - |
| Purified water | | Bal. | Bal. | Bal. | Bal. |
| Total amount | | 100 ml | 100 ml | 100 ml | 100 ml |
| pH | | 7.46 | 7.45 | 7.45 | 7.48 |
| Osmotic pressure (mOsm) | | 293 | 305 | 293 | 305 |
| Viscosity (mPa-s) | | 1.16 | 1.11 | 1.14 | 1.11 |
| Coloration of solution | | None | None | None | None |
| Hydrogel contact lens applied | | 1 | 1 | 1 | 1 |
| Friction coefficient (Ave.) | 1 | 0.07 | 0.88 | 0.51 | 0.40 |
| | 30 | 0.13 | 1.21 | 0.67 | 0.68 |
| Surface smoothness | | ++ | - | - | - |
| Durability of surface smoothness | | ++ | | | |

**[Table 2]**

| | | Examples | | | | | | | | | | | Comparative Examples | | | |
|---|---|---|---|---|---|---|---|---|---|---|---|---|---|---|---|---|
| | | 8 | 9 | 10 | 11 | 12 | 13 | 14 | 15 | 16 | 17 | 18 | 4 | 5 | 6 | 7 |
| Surfactant (w/v%) | P407 | 0.05 | 0.05 | 0.05 | 0.05 | 0.05 | 0.05 | - | - | - | - | - | - | 0.05 | - | - |
| | P-85 | - | - | - | - | - | - | 0.05 | - | - | - | - | - | - | - | - |
| | F-108 | - | - | - | - | - | - | - | 0.05 | - | - | - | - | - | - | - |
| | F-68 | - | - | - | - | - | - | - | - | 0.05 | - | - | - | - | - | - |
| | HCO-60 | - | - | - | - | - | - | - | - | - | 0.05 | - | - | - | - | - |
| | TR-704 | - | - | - | - | - | - | - | - | - | - | 0.05 | - | - | - | - |
| Polysaccharide (w/v%) | Alginic acid | 0.1 | 0.1 | 0.1 | 0.1 | 0.1 | 0.1 | - | - | - | - | 0.1 | - | - | 0.1 | 0.1 |
| | Hyaluronic acid | - | - | - | - | - | - | 0.1 | - | - | - | - | - | - | - | - |
| | Sodium chondroitin sulfate | - | - | - | - | - | - | - | 0.1 | - | - | - | - | - | - | - |
| | HPMC | - | - | - | - | - | - | - | - | 0.1 | - | - | - | - | - | - |
| | Pullulan | - | - | - | - | - | - | - | - | - | 0.1 | - | - | - | - | - |
| Buffering agent (w/v%) | Boric acid | 0.99 | - | 0.5 | 0.5 | 0.5 | 0.99 | 0.99 | 0.99 | 0.99 | 0.99 | 0.99 | 0.99 | 0.99 | 0.99 | - |
| | Borax | 0.38 | - | 0.19 | 0.19 | 0.19 | 0.38 | 0.38 | 0.38 | 0.38 | 0.38 | 0.38 | 0.38 | 0.38 | 0.38 | - |
| | Sodium hydrogen phosphate | - | 0.12 | - | - | - | - | - | - | - | - | - | - | - | - | 0.12 |
| | Potassium dihydrogen phosphate | - | 0.10 | - | - | - | - | - | - | - | - | - | - | - | - | 0.1 |
| Isotonicity agent (w/v%) | Sodium chloride | 0.18 | 0.80 | 0.35 | 0.50 | - | 0.30 | 0.30 | 0.30 | 0.30 | 0.30 | 0.30 | 0.30 | 0.30 | 0.30 | 0.80 |
| | Potassium chloride | 0.16 | 0.02 | - | - | - | - | - | - | - | - | - | - | - | - | 0.02 |
| | Polypropylene glycol | - | - | 0.57 | - | 1.30 | - | - | - | - | - | - | - | - | - | - |
| Chelating agent (w/v%) | Ethylenediaminetetraacetic acid | - | 0.05 | 0.05 | 0.05 | 0.05 | 0.05 | 0.05 | 0.05 | 0.05 | 0.05 | 0.05 | 0.05 | 0.05 | 0.05 | 0.05 |
| | Citric acid | 0.03 | - | - | - | - | - | - | - | - | - | - | - | - | - | - |
| 1N HCl (µL) | | - | - | - | - | - | - | 0.65 | 0.65 | 0.65 | 0.65 | - | 0.65 | 0.65 | - | - |
| 1N NaOH (µL) | | - | - | - | - | - | - | - | - | - | - | - | - | - | - | - |
| Purified water | | Bal. | Bal. | Bal. | Bal. | Bal. | Bal. | Bal. | Bal. | Bal. | Bal. | Bal. | Bal. | Bal. | Bal. | Bal. |
| Total amount | | 100 ml | 100 ml | 100 ml | 100 ml | 100 ml | 100 ml | 100 ml | 100 ml | 100 ml | 100 ml | 100 ml | 100 ml | 100 ml | 100 ml | 100 ml |
| pH | | 7.40 | 7.21 | 7.44 | 7.47 | 7.46 | 7.46 | 7.45 | 7.45 | 7.45 | 7.45 | 7.45 | 7.45 | 7.45 | 7.46 | 7.17 |
| Osmotic pressure (mOsm) | | 293 | 291 | 302 | 271 | 286 | 292 | 305 | 305 | 305 | 305 | 305 | 305 | 305 | 292 | 291 |
| Coloration of solution | | None | None | None | None | None | None | None | None | None | None | None | None | None | None | None |
| Hydrogel contact lens applied | | 1 | 1 | 1 | 1 | 1 | 1 | 1 | 1 | 1 | 1 | 1 | 1 | 1 | 1 | 1 |
| Friction coefficient (Ave.) | 1 | 0.02 | 0.16 | 0.05 | 0.25 | 0.16 | 0.02 | 0.17 | 0.06 | 0.16 | 0.09 | 0.08 | 1.17 | 0.15 | 0.77 | 0.80 |
| | 30 | 0.08 | 0.38 | 0.27 | 0.39 | 0.38 | 0.20 | 0.45 | 0.38 | 0.54 | 0.17 | 0.14 | 1.66 | 0.87 | 1.12 | 0.89 |
| Surface smoothness | | ++ | + | ++ | + | + | ++ | + | ++ | + | ++ | ++ | - | + | - | - |
| Durability of surface smoothness | | ++ | ++ | ++ | ++ | ++ | ++ | + | + | + | ++ | ++ | | - | | |

**[Table 3]**

| | | Examples | | Comparative Examples | | | | |
|---|---|---|---|---|---|---|---|---|
| | | 19 | 20 | 8 | 9 | 10 | 11 | 12 |
| Surfactant (w/v%) | P407 | 0.05 | 0.05 | 0.05 | 0.05 | - | - | - |
| Polysaccharide (w/v%) | Alginic acid | 0.1 | 0.1 | - | - | 0.1 | 0.1 | - |
| Buffering agent (w/v%) | Boric acid | 0.99 | - | 0.99 | - | 0.99 | - | 0.99 |
| | Borax | 0.38 | - | 0.38 | - | 0.38 | - | 0.38 |
| | Na₂HPO₄ | - | 0.24 | - | 0.24 | - | 0.24 | - |
| | NaH₂PO₄·2H₂O | - | 0.05 | - | 0.05 | - | 0.05 | - |
| Isotonicity agent (w/v%) | Sodium chloride | 0.3 | 0.83 | 0.3 | 0.83 | 0.3 | 0.83 | 0.3 |
| | Potassium chloride | - | - | - | - | - | - | - |
| | Polypropylene glycol | - | - | - | - | - | - | - |
| Chelating agent (w/v%) | Ethylenediaminetetraacetic acid | 0.05 | 0.05 | 0.05 | 0.05 | 0.05 | 0.05 | 0.05 |
| | Citric acid | - | - | - | - | - | - | - |
| 1N HCl (µL) | | - | - | 0.65 | - | - | - | 0.65 |
| 1N NaOH (µL) | | - | 0.5 | - | 0.5 | - | - | - |
| Purified water | | Bal. | Bal. | Bal. | Bal. | Bal. | Bal. | Bal. |
| Total amount | | 100 ml | 100 ml | 100 ml | 100 ml | 100 ml | 100 ml | 100 ml |
| Hydrogel contact lens applied | | 2 | 2 | 2 | 2 | 2 | 2 | 2 |
| Friction coefficient (Ave.) | 1 | 0.07 | 0.06 | 0.19 | 0.51 | 0.33 | 0.43 | 1.30 |
| | 30 | 0.12 | 0.16 | 0.50 | 0.56 | 0.49 | 0.82 | 1.42 |
| Surface smoothness | | ++ | ++ | + | - | - | - | - |
| Durability of surface smoothness | | ++ | ++ | - | | | | |

P407: Polyoxyethylene (196) polyoxypropylene (67) glycol
P-85: Polyoxyethylene (50) polyoxypropylene (40) glycol
F-108: Polyoxyethylene (300) polyoxypropylene (55) glycol
F-68: Polyoxyethylene (160) polyoxypropylene (30) glycol
HCO-60: Polyoxyethylene hardened castor oil 60
TR-704: Ethylenediamine tetrapolyoxyethylene polyoxypropylene
Borate buffer: boric acid, borax, sodium chloride, edetic acid
Phosphate buffer: sodium hydrogen phosphate, sodium dihydrogen phosphate, sodium chloride
HPMC: Hydroxypropyl methylcellulose
Pullulan: PULLULAN PI-20

### Industrial applicability

The contact lens solution and method according to one embodiment of the present invention can impart surface smoothness to a contact lens thereby reducing friction between the contact lens and ocular tissues occurring when wearing and blinking. As a result, the contact lens solution and method according to one embodiment of the present invention are expected to be used for the purposes of suppressing or alleviating the fracturing of the tear fluid layer and the development of ocular disorders, and comfortably wearing a contact lens.

### Cross-reference of related applications

The present application claims the benefit of priority to Japanese Patent Application No. 2020-131547, filed on August 3, 2020, the disclosure of which is incorporated herein by reference in its entirety.

## Claims

1. A contact lens solution comprising a nonionic surfactant and a polysaccharide.

2. The contact lens solution according to claim 1, further comprising at least one component selected from the group consisting of a buffering agent, an isotonicity agent and a chelating agent.

3. The contact lens solution according to claim 1 or 2, wherein the contact lens solution is a contact lens solution for smoothing the surface of a contact lens.

4. The contact lens solution according to any one of claims 1 to 3, wherein the nonionic surfactant is at least one nonionic surfactant selected from the group consisting of polyoxyethylene-based surfactants, and/or the polysaccharide is at least one polysaccharide selected from the group consisting of mucopolysaccharides and cellulose-based polymer compounds.

5. The contact lens solution according to any one of claims 1 to 4, wherein the mass ratio of the nonionic surfactant and the polysaccharide (nonionic surfactant:polysaccharide) is 1:0.01 to 1:50 and/or the total content of the nonionic surfactant and the polysaccharide is 0.001 w/v% to 5.0 w/v% relative to the total amount of the solution.

6. The contact lens solution according to any one of claims 2 to 5, wherein the ratio ([B]:[A]) of the total content ([B]) of the buffering agent, the isotonicity agent and the chelating agent relative to the total content ([A]) of the nonionic surfactant and the polysaccharide is 1:0.1 to 1:100.

7. The contact lens solution according to any one of claims 1 to 6, wherein the contact lens solution is a contact lens package solution or a contact lens care solution.

8. A method for smoothing the surface of a contact lens, comprising bringing a contact lens into contact with the contact lens solution according to any one of claims 1 to 7.

9. A method of producing a contact lens having surface smoothness, comprising bringing a contact lens into contact with the contact lens solution according to any one of claims 1 to 7 to obtain the contact lens having surface smoothness.
